# EUROPEAN PATENT APPLICATION

(11) **EP 3 056 147 A1**
(43) Date of publication of application: **17.08.2016**
(21) Application number: 14851686.7
(22) Date of filing: 17.09.2014
(51) Int. Cl.: A61B 5/151

(54) **BLOOD-COLLECTING NEEDLE HAVING VARIABLE LENGTH AND PROVIDED WITH PAIN, INFLAMMATION, AND RISK PREVENTION FUNCTIONS**

(30) Priority: 11.10.2013 KR 20130121594
(71) Applicant: Kim, Kwang-il, Daejeon 300-700 (KR)
(72) Inventor: Kim, Kwang-il, Daejeon 300-700 (KR)
(74) Representative: Loo, Chi Ching
(86) International application number: PCT/KR2014/008631
(87) International publication number: WO 2015/053490

(57) **Abstract**

The present invention relates to a blood-collecting needle having a variable length and provided with pain, inflammation, and risk prevention functions and, more particularly, to a blood-collecting needle in which, when a disposable blood-collecting needle is inserted into a semiautomatic blood collection device to collect blood, the blood-collecting needle not only reduces pain, but achieves perfect sanitation to relieve the sense of danger and anxiety felt by a user, and the length of the blood-collecting needle is freely changed in multiple stages. According to the present invention, a multi-bump disposed on a lower end of a main body may smoothly pass through a needle passage of the blood collection device to reduce pain while instantly contacting the peripheral epidermis during self-injection. The multi-bump may sanitarily collect blood while blocking the needle passage of the blood collection device. Also, the length of the blood-collecting needle may be freely changed in an optimal state according to various conditions by means of a simple method in which the multi-bump is cut in multiple stages.

## Description

### CROSS-REFERENCE TO RELATED APPLICATION

This application claims under 35 U.S.C. §119(a) the benefit of priority to Korean Patent Application No. 10-2013-0121594 filed on October 11, 2013, the entire contents of which are incorporated herein by reference.

### BACKGROUND

### (a) Technical Field

The present invention relates to a technology which is able to alleviate any pain when blood is collected by inserting a disposable blood-collecting needle into a semiautomatic blood-collecting device, and a sanitation can be improved, and any risk or anxiety that a user may feel can be eliminated, and the length of a needle can be freely changed.

### (b) Background Art

When it needs to collect a small amount of blood so as to measure the level of a blood sugar in case of diabetic or test a blood type, we, in general, use a disposable blood-collecting needle (a lancet's needle) which is engaged to a blood-collecting device (a lancet device).

Moreover, when blood is collected by a typical method, the blood, in general, is collected from a vein in a dermis at a depth of below 1 mm by inserting a needle into up to an inner skin portion of a first joint of a finger.

As a prior art technology related with the aforementioned disposable blood-collecting needle, there is a Korean patent registration number 1272621 which was invented by the same applicant as the present invention and was registered as a patent in Korea, the title of which is a disposable blood-collecting needle having a variable needle length.

The aforementioned prior art technology may allow to provide an automated manufacturing process when assembling a blood-collecting needle. Since the length of the blood-collecting needle can be freely adjusted to the optimum state, the above technology is a very useful invention.

The whole configurations of the aforementioned prior art technology, however, is very complicated, and it needs an additional process due to increased assembling works. For this reason, the increased price of the blood-collecting needle due to the increased manufacturing cost may be a burden to a patient or a customer. Moreover, a gap may be inevitably formed between a needle and a nut adapted to adjust the length of the needle during the collection of blood, which may cause a sanitation problem.

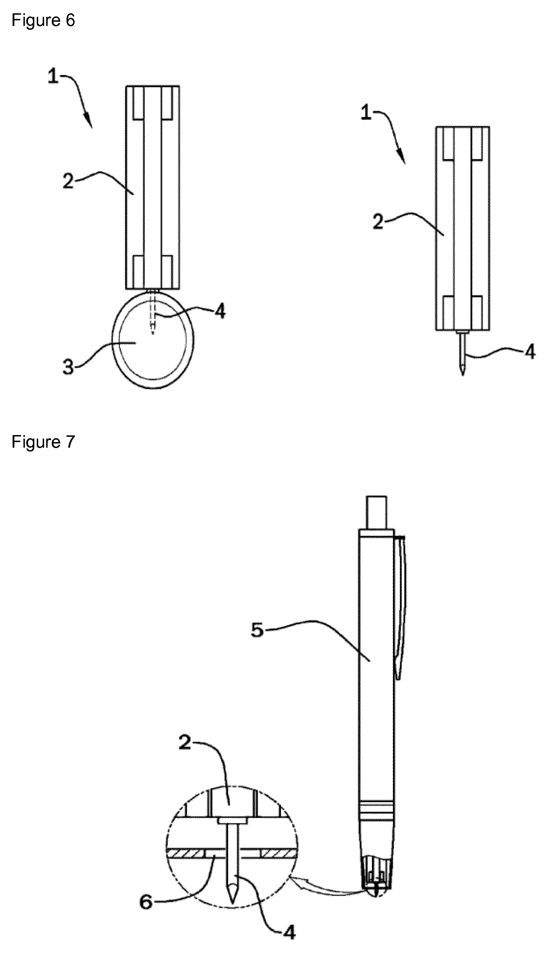

The disposable blood-collecting needle 1 as in Figure 6 which is being usually used may cause any infection and risk, but it is convenient to use and is cheap, whereupon many users are usually using the aforementioned needle.

As illustrated in Figures 6 and 7, the conventional disposable blood-collecting needle 1 is used after a needle protection part 3 has been removed from a body 2. In this case, the exposing length of the blood-collecting needle 4 may become too long, for which risk and anxiety increase, and a severe pain due to the long needle may be accompanied during the collection of blood.

Moreover, a predetermined gap may be formed between a needle passage 6 formed at the blood-collecting device 5, and the blood-collecting needle 4 just before the needle is inserted into the epidermis when collecting blood using the disposable blood-collecting needle 4. The blood may be contaminated between the blood-collecting device 5 and the blood-collecting needle via the aforementioned gap.

### SUMMARY OF THE DISCLOSURE

The present invention has been made in an effort to solve the above-described problems associated with a conventional disposable blood-collecting needle.

It is an object of the present invention to provide a blood-collecting needle having a variable length and provided with a pain, inflammation, and risk prevention functions, wherein any pain can be alleviated in such a way that a multi-bump formed at a lower end of a body smoothly passes through a needle passage of a blood-collecting device and instantly comes into contact with a surrounding epidermis when giving an injection, and the multi-bump blocks a gap between the needle and the needle passage of the blood-collecting device while passing through the needle passage of the blood-collecting device, by means of which a sanitary blood-collecting can be carried out.

Moreover, the length of the needle can be freely changed depending on a user's skin elasticity in such a simple way that the multi-bump is formed cut-away to form multiple steps.

To achieve the above objects, there is provided a blood-collecting needle having a variable length and provided with a pain, inflammation, and risk prevention function wherein a first multi-bump is integrally protruding from a lower end portion of a body of a blood-collecting needle, and a second multi-bump is integrally protruding from a lower end portion of the first multi-bump, and a needle which is inserted inside the body, passes through the center of each of the first and second multi-bumps and is partially exposing to the outside.

In the present invention, a cut-away groove is formed concave at an outer circumferential surface of an upper portion of the first multi-bump and at an outer circumferential surface of an upper portion of the second multi-bump.

### ADVANTAGEOUS EFFECTS

According to the present invention, since the multi-bump instantly comes into contact with the surrounding epidermis like hitting the same when giving an injection, thus distributing and alleviating any pain, and the multi-bump is able to instantly expand the surrounding portions of an injection spot at the same time the needle passes through the epidermis, thus allowing the needle to be smoothly inserted, by means of which a reliable blood-collecting can be carried out even though the exposing length of the needle is short.

Moreover, since the needle insertion is completed in a state where the multi-bump is blocking all the gaps between the needle and the needle passage during the collection of blood, a sanitary blood collection can be carried out irrespective of any contamination of the blood-collecting device.

Furthermore, according to the present invention, since the blood collection can be reliably carried out even though the exposing length of the needle is very short, a severe pain does not occur, and the user won't feel any risk or anxiety during the collection of blood. In case of children, they may not have any trauma with respect to the collection of blood.

In addition, since the multi-bump is partially cut-away using the cut-away groove formed at the multi-bump, the length of the needle can be adjusted in multiple stages depending on given conditions, whereby the collection of blood can be smoothly carried out, and the applicability of products can be improved.

### BRIEF DESCRIPTION OF THE DRAWINGS

The above and other features of the present invention will now be described in detail with reference to certain exemplary embodiments thereof illustrated the accompanying drawings which are given hereinbelow by way of illustration only, and thus are not limitative of the present invention, and wherein:
Figure 1 is a front view illustrating a disposable blood-collecting needle according to the present invention;
Figure 2 is a front view illustrating a disposable blood-collecting needle from which a needle protection part is removed, according to the present invention;
Figure 3 is an enlarged cross sectional view illustrating a state wherein a blood collection is being carried out in a state where a disposable blood-collecting needle is engaged to a blood-collecting device according to the present invention;
Figure 4 is a view illustrating a state where a one-stage multi-bump is partially cut-away according to the present invention;
Figure 5 is a view illustrating a state where a two-stage multi-bump is partially cut-away according to the present invention;
Figure 6 is a front view illustrating a conventional disposable blood-collecting needle while illustrating a state where a needle protection part is removed; and
Figure 7 is a view illustrating a state where a conventional disposable blood-collecting needle is engaged to a blood-collecting device.

It should be understood that the appended drawings are not necessarily to scale, presenting a somewhat simplified representation of various preferred features illustrative of the basic principles of the invention. The specific design features of the present invention as disclosed herein, including, for example, specific dimensions, orientations, locations, and shapes will be determined in part by the particular intended application and use environment.

In the figures, reference numbers refer to the same or equivalent parts of the present invention throughout the several figures of the drawing.

### DETAILED DESCRIPTION

The preferred embodiments of the present invention to implement the objects of the present invention will be described.

The whole configuration according to a preferred embodiment of the present invention will be described. The blood-collecting needle having a variable length and provided with a pain, inflammation, and risk prevention function according to the present invention may include, but is not limited to, a body 10, a first multi-bump 20, a second multi-bump 30, a blood-collecting needle 40, and a needle protection part 50.

The present invention will be described in more detail for the sake of easier implementations.

The body 10 of the present invention is used in a state where a predetermined portion thereof is inserted in a hitting member installed inside of a typical blood-collecting device 5. The first multi-bump 20 is integrally protruding from in the center of a lower portion of the body 10, wherein the first multi-bump 20 is equipped with various functions as major features of the present invention.

Moreover, the second multi-bump 30 having a smaller diameter than that of the first multi-bump 20 is integrally protruding from in the center of a lower portion of the first multi-bump 20, wherein the second multi-bump 30 is formed integrally stepped in order for the length of the blood-collecting needle 40 to be changed in multiple stages.

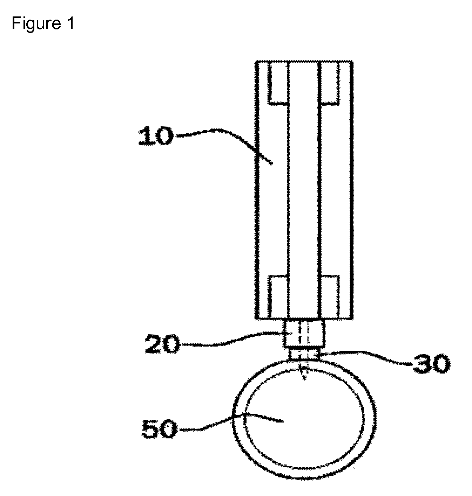

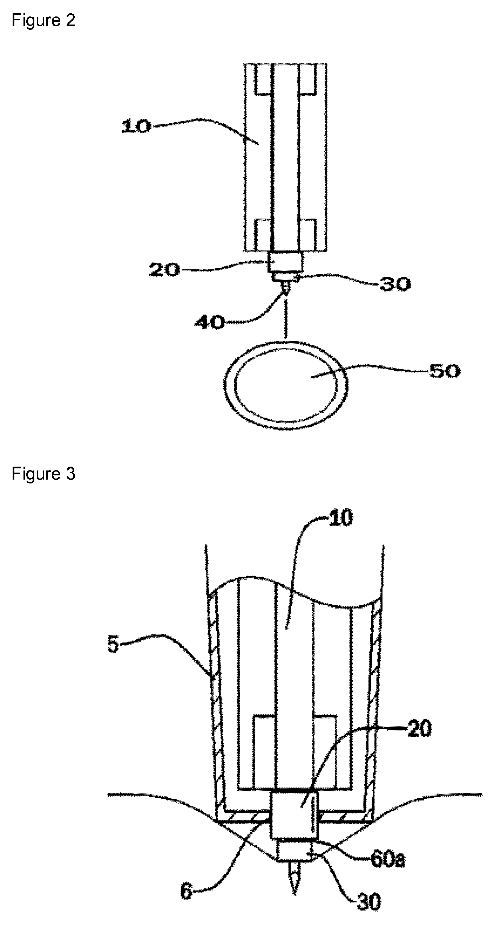

As illustrated in Figures 1 and 2, the blood-collecting needle 40 is inserted integral inside of the body 10, and one end of the blood-collecting needle 40 passes through the center of each of the first and second multi-bumps 20 and 30 and is exposing to the outside.

Since a partial part of the blood-collecting needle 40 passes through the insides of the first and second multi-bumps 20 and 30, the length thereof exposing to the outside after the needle protection part 50 has been removed is much shorter than that of the conventional blood-collecting needle 4 as illustrated in Figure 2.

The reason why a reliable blood collection can be carried out even though the exposing length of the blood-collecting needle 40 is much shorter than that of the conventional blood-collecting needle is that a needle insertion can be carried out while the surrounding portions of the injection spot are being expanded with the aid of the second multi-bump 30 during the collection of blood.

In the present invention, if an end of the blood-collecting needle 40 comes into contact with the skin during the collection of blood, the skin may be formed in a V-shape about the needle due to the resistance of epidermis. Here, the second multi-bump 30 comes into contact with the skin at the edges of the V-shape, and the blood-collecting needle 40 passes through the epidermis, and at the same time the skin near the injection spot is expanded, thus allowing the needle to be easily inserted, by means of which the needle insertion can be smoothly carried out. In this way, any pain can be greatly alleviated. Since the short needle is inserted, the pain can be alleviated, while lowering risks.

Moreover, since the needle insertion is carried out in a state where the skin near the injection spot is expanded again with the aid of the second multi-bump 30, the skin can be quickly contracted and return back to its initial state immediately after the blood collection is finished. For this reason, a skin recovery is very fast.

The second multi-bump 30 is able to a special effect on distribution and alleviation of pain since it instantly comes into contact with the epidermis of the surrounding of the injection spot at the time of injection. This may be like a doctor instantly hits an injection spot with a hand at the time of injection or a balloon can pop more easily by a needle peak if it becomes larger.

Moreover, as illustrated in Figure 3, the first multi-bump 20 is adapted to allow a sanitary blood collection without any potential infection irrespective of the contamination of the blood-collecting device 5 since a needle insertion is completed in a state where all the gaps between the blood-collecting needle 40 and the needle passage 6 formed at a lower portion of the blood-collecting device 5. In particular, since a reliable blood collection is available even though the exposing length of the blood-collecting needle 40 is short, by means of which any pain, risk and anxiety can be eliminated during the injection.

The needle protection part 50 is disposed covering the outer circumference of the blood-collecting needle 40 which is exposing to the outsides of the first and second multi-bumps 20 and 30. The aforementioned needle protection part 50 is connected integral to a lower portion of the second multi-bump 50.

The needle protection part 50 may allow to provide a sanitary environment without any risk with respect to a bacterium infection by providing an asepsis environment while protecting the blood-collection needle 40. The needle protection part 50 is also able to prevent any transformation of the blood-collecting needle 40. Only when the blood-collecting needle 40 is actually used, the needle protection part 50 is removed from the second multi-bump 30, whereupon the blood-collecting needle 40 can be used in an asepsis condition. When a blood collection is carried out with the needle protection part 50 having removed, the second multi-bump 30 passes through the needle passage 6 formed at a lower portion of the blood-collecting device 5 and is exposing to the outside, so the second multi-bump 30 can instantly come into contact with the epidermis near the injection spot when inserting the blood-collecting needle 40. At this time, the length of the blood-collecting needle 40 is fixed at a basic first stage where the length thereof is as shortest as possible, which is long enough not to interrupt with the blood collection as compared to the conventional disposable blood-collecting needle.

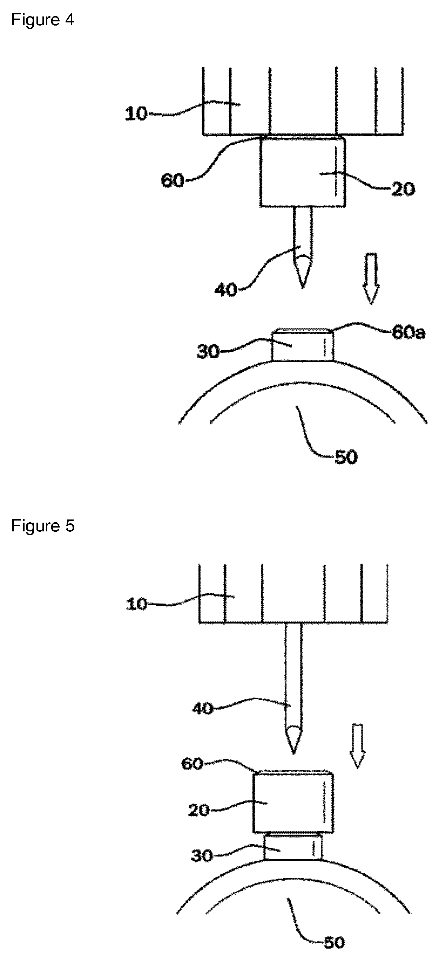

Moreover, when a longer needle is necessary since the blood collection portion is thick, a blood collection effect is bad due to a weak hitting force of the blood collection device, or a blood collection should be carried out from a deeper portion, as illustrated in Figure 4, the second multi-bump 30 can be separated from the first multi-bump 20 and can be used in an asepsis state. The first multi-bump 20 may pass through the needle passage 6 formed at a lower portion of the blood-collecting device 5 and may expose to the outside, whereby the first multi-bump 20 can instantly come into contact with the epidermis near the injection spot when inserting the blood-collecting needle 40. Here, the exposing length of the blood-collecting needle 40 is fixed at an adjusted two-stage.

If the disposable blood-collecting needle is intended to be used alone without using any blood-collecting device or the needle the length of which is same as that of the conventional disposable blood-collecting needle is necessary for the sake of blood collection from a deeper portion, the blood-collecting needle of the present invention may be used in a state where the first multi-bump 20 is removed from the body 10, and the exposing length of the blood-collecting needle 40 is fixed at an adjusted third-stage. In this case, the longest needle is available, but the pain, infection and risk prevention functions which are the advantageous features of the present invention are not available.

In the present invention, the needle protection part 50 can be readily removed in such a way to rotate the needle protection part 50, and the first and second multi-bumps 20 and 30 can be separated using a predetermined knife-like blade. For the sake of easier separations, cut-away grooves 60 and 60a may be formed at an outer circumference of the upper portion of each of the first and second multi-bumps 20 and 30.

The first and second multi-bumps 20 and 30 may be cut away in multiple stages in such a way that the cut-away grooves 60 and 60a may be incised partially at regular intervals. In this way, the length of the blood-collecting needle 40 exposing to the outside can be available, whereupon the blood collection can be reliably carried out while changing the length of the needle to the optimum length under any condition.

Moreover, if the needle protection part 50 is removed after an incision line has been made at the circumferences of the cut-away grooves 60 and 60a so as to select a predetermined length of the needle, the removing portions of the first and second multi-bumps 20 and 30 attached to the needle protection part 50 will be simultaneously removed. If the first and second multi-bumps 20 and 30 are cut so as to change the length of the needle during a blood collection, the separated removing portions of the first and second multi-bumps 20 and 30 are slightly pushed using a tip of a knife-like blade and are removed.

The invention has been described in detail with reference to preferred embodiments thereof. However, it will be appreciated by those skilled in the art that changes may be made in these embodiments without departing from the principles and spirit of the invention, the scope of which is defined in the appended claims and their equivalents.

### Legend of Reference Numbers

1: Disposable blood-collecting needle
2: Body
3, 50: Needle protection part
4, 40: Blood-collecting needle
5: Blood-collecting device
6: Needle passage
20: First multi-bump
30: Second multi-bump
60, 60a: Cut-away grooves

## Claims

1. A blood-collecting needle having a variable length and provided with a pain, inflammation, and risk prevention functions, comprising:
a first multi-bump which is formed protruding from in the center of a lower portion of a body;
a second multi-bump which is formed protruding from in the center of a lower portion of the first multi-bump;
a blood-collecting needle which is inserted in the body, wherein an end of the blood-collecting needle passes through the center of each of the first and second multi-bumps and is exposing to the outside; and
a needle protection part which is connected integral to a lower end portion of the second multi-bump, while covering the outer circumference of the blood-collecting needle which is exposing to the outsides of the first and second multi-bumps.

2. The needle of claim 1, wherein when the blood-collecting needle is used, the blood-collecting needle can be used in an asepsis state in such a way to remove the needle protection part from the second multi-bump, and during the blood collection, the second multi-bump is configured to pass through a needle passage formed at a lower end portion of the blood-collecting device and expose to the outside, and the length of the blood-collecting needle can be changed to a first stage.

3. The needle of claim 1, wherein when the blood-collecting needle is used, the blood-collecting needle can be used in an asepsis state in such a way to remove the second multi-bump from the first multi-bump, and during the blood collection, the first multi-bump is configured to pass through the needle passage formed at a lower end portion of the blood-collecting device and expose to the outside, and the length of the blood-collecting needle can be changed to a second stage.

4. The needle of claim 1, wherein when the blood-collecting needle is used, the blood-collecting needle can be used in an asepsis state in such a way to remove the first multi-bump from the body, and the length of the blood-collecting needle is changed to a third stage.

5. The needle of claim 1, wherein cut-away grooves are formed concave at an outer circumference surface of an upper portion of the first multi-bump and an outer circumference surface of the second multi-bump, respectively.
